# EUROPEAN PATENT SPECIFICATION

(11) **EP 3 919 054 B1**
(45) Date of publication and mention of the grant of the patent: **14.06.2023**
(21) Application number: 21177871.7
(22) Date of filing: 04.06.2021
(51) Int. Cl.: A61K 31/351, A61K 36/28, A61K 38/06, A61K 36/82, A61K 31/198, A61K 31/60, A61K 31/6615, A61P 17/10

(54) **PHYTOTHERAPEUTIC COMPOSITIONS FOR TOPICAL OR ORAL USE IN ACNE TREATMENT**
PHYTOTHERAPEUTISCHE ZUSAMMENSETZUNGEN FÜR DIE TOPISCHE ODER ORALE ANWENDUNG IN DER BEHANDLUNG VON AKNE
COMPOSITIONS PHYTOTHERAPEUTIQUES POUR LE TRAITEMENT DE L'ACNÉ POUR UTILISATION PAR VOIE LOCALE OU ORALE

(30) Priority: 05.06.2020 IT 202000013393
(43) Date of publication of application: 08.12.2021
(73) Proprietor: Sestito, Francesco, 30034 Mira (VE) (IT); Giovannone, Daniele, 03100 Frosinone (FR) (IT); Vianello, Dino, 31021 Mogliano Veneto (TV) (IT)
(72) Inventor: Sestito, Francesco, 30034 Mira (VE) (IT); Giovannone, Daniele, 03100 Frosinone (FR) (IT); Vianello, Dino, 31021 Mogliano Veneto (TV) (IT)
(74) Representative: Di Bernardo, Antonio

(56) References cited:
- JP-B2- 6 505 905
- US-A- 5 904 924
- US-B2- 9 561 198
- CHANDIL NIDHI ET AL: "Comparison of Metformin andNAcetylcysteine on Clinical, Metabolic Parameter and Hormonal Profile in Women with Polycystic Ovarian Syndrome", THE JOURNAL OF OBSTETRICS AND GYNECOLOGY OF INDIA, SPRINGER INDIA, INDIA, vol. 69, no. 1, 28 May 2018 (2018-05-28), pages 77-81, XP036691368, ISSN: 0971-9202, DOI: 10.1007/S13224-018-1135-3 [retrieved on 2018-05-28]

## Description

### TECHNICAL FIELD OF THE INVENTION

The present invention describes a formulation adapted to mitigate the effects of acne and to limit the persistence thereof. This formulation comprises phytotherapeutic preparations combined between them according to the presence of specific active ingredients in an adequate proportion to give a high biological response.

### Background

Acne is a localised inflammation of the sebaceous glands at the base of the follicles which typically develops during or after puberty, under hormonal stimulation, and is associated with an excessive proliferation of microorganisms normally present on the skin. Acne usually manifests itself with the formation of pustules and/or papules and is localised in particular on the skin of the face and neck; it is generally transitory but can persist for a long time, even for several years. The presence of acne is often associated with psychological distress and possibly permanent physical damage, such as small scars.

The typical treatment for acne consists mainly of the topical application of retinoidbased products, the action thereof possibly reinforced by topical application of benzoyl peroxide. Often anti-inflammatory drugs are also associated; antibiotics may also be used, either topically or systemically. In some cases, hormone therapy may also be indicated.

All these drugs have moderate efficacy on acne, which often persists for a long time, and have important side-effects, which may lead the patient to disregard the indicated dosage or stop treatment. In addition, some patients have a strong aversion to the use of drugs in general and prefer using phytotherapeutic remedies, which are perceived as more natural.

The many phytotherapeutic products traditionally used include for example tea *(Camellia sinensis,* leaf) chamomile *(Matricaria chamomilla,* flower), witch hazel, marigold and many others, all of dubious or moderate efficacy.

In addition to the traditional use of the plant drug, e.g., in herbal teas, modern phytotherapy uses extracts, which are concentrated forms of the active plant ingredients. Typically, a concentration ratio of the extract to the original drug is indicated, but in some cases the extracts are titrated and standardised by content of the active ingredients; these extracts are particularly useful in topical preparations. However, it is not always possible to identify all the active compounds present in a phytotherapeutic preparation and to attribute a biological effect to each of them: moreover, bioactive substances present at the same time may interact with each other with both synergistic and antagonistic effects.

### Summary of the invention

The invention comprises a mixture of three active ingredients, of which two plant drugs, mixed in defined proportions; said mixture is used for the preparation of creams, ointments, lotions and other formulations for topical and/or for oral use and is useful in the treatment of acne. A surprising synergistic effect has been found in the treatment of acne among a number of active ingredients of herbal and synthetic origin, all of which are already known but never used in combination for acne therapy. In a particular embodiment, local treatment can be combined with oral administration of one or more of the active ingredients.

The composition is more effective than that of the single components both in reducing inflammation and in reducing sebaceous secretion, and it also has satisfactory antibacterial activity against *Propionibacterium acne* (now renamed *Cutibacterium acne).* In one embodiment, plant drugs may be present in the form of extracts, e.g., fluid or dry extract or tincture. In a preferred embodiment, the composition uses ingredients in the form of a pure active ingredient or of a plant extract with a known title in active ingredients.

In a special embodiment, the active ingredients of said composition are also administered orally, as well as by local application.

### Detailed description of the invention

One active is epigallocatechin-3-gallate (EGCG), an ester of gallic acid with epigallocatechin, a polyphenol found in tea leaves (*Camellia sinensis*) associated with other similar catechins; by oral administration it is known as an antioxidant and for reducing cholesterol. EGCG is applied in acne therapy for its anti-inflammatory properties and for the reduction of sebum secretion: see e.g., Yoon et al., J Investigative Dermatology no. 133, page 429-440, 2013. Similarly, less pure preparations, commercially referred to as "green tea extract", in which EGCG is associated with other substances similar in chemical structure and biological effect, such as epicatechin, gallocatechin and their esters with gallic acid, can also be applied; such extracts can have a very high polyphenol content, e.g., 90-95%. In tea extracts, the catechins listed above are present in widely varying proportions, but EGCG is always the most abundant catechin. There is no real synergy between the various catechins; pure EGCG is essentially as effective as the extracts. The use of both pure EGCG and concentrated tea extracts in the formulations of preparations for topical use is hampered by their limited solubility; they are typically applied in amounts around 0.1%. They can of course also be taken orally, but (unless associated with topical preparations) orally they are not effective in treating acne.

A second phytotherapeutic active present in the composition is burdock (*Arctium lappa*), generally used in cosmetics for the anti-inflammatory and calming effect thereof. It is not considered effective in the specific treatment of acne but rather as an anti-wrinkle and in order to obtain a skin-rejuvenating effect. A detailed description of the effects of burdock fruit extract can be found for example in A. Knott et al, J Cosmetic Dermatology 7, 281-9, 2008. The combination of EGCG and burdock in topical preparations provides a moderate anti-inflammatory action without affecting the other aspects of acne; however, these are palliative preparations and do not remedy the presence of acne. US 9561198 B2 describes treatment of acne with topical skin care compositions that include Arctium lappa (Burdock) root extract, optionally with green tea extract or acetyl cysteine.

A third characteristic active ingredient of our composition is glutathione, a tripeptide normally present in mammalian cells, where it plays a role in detoxifying from heavy metals and various metabolites. Glutathione can be found in a reduced form, generally indicated by the abbreviation GSH, or in an oxidised form, referred to as GSSG: for the purposes of the efficacy in our composition, the forms thereof (oxidised or reduced) and the other biological sources, whether prophylactics or precursors, are equivalent. Glutathione and the sources thereof are substances that are ineffective in themselves in the treatment of acne, unless associated with the active ingredients described above, and particularly with EGCG. No antimicrobial activity is known, particularly towards *P. acne* or other microorganisms typically found on the skin. In topical applications, glutathione can be used both in its reduced form and in its oxidised form, which is particularly stable. In a particular embodiment, fat-soluble glutathione derivatives can be used, which are particularly suitable for topical formulations. For example, WO2012/134758 describes the topical use of S-palmitoyl-glutathione for the reduction of skin wrinkles: however, no efficacy in the treatment of acne is described.

In a further embodiment, topical therapy can be combined with oral therapy: both glutathione and the precursors thereof, such as for example L-cysteine or N-acetyl-L-cysteine, are particularly suitable for oral administration.

In a further embodiment, the efficacy of the topical composition is further enhanced when an acid agent with exfoliating and/or chelating properties, such as for example salicylic acid, tartaric acid, phytic acid and their salts, is added to the active phytotherapeutic products.

In a further embodiment, a suitable dye can be added to the topical formulation, obtaining a coloured cream with different shades of colour. In this case, a cosmetic preparation with covering powers is obtained, improving the appearance of the face; this makes it possible to immediately mitigate the psychological effects of acne, while maintaining good effectiveness in the actual treatment. The colouring of the formulation also allows better adherence to therapy, as patients are encouraged to apply the topical composition regularly.

The invention is illustrated by the following examples.

### Example 1 Preparation of formulations for topical application

To test the efficacy of the single ingredients and their combinations, model formulations were prepared using the minimum number of excipients capable of forming a cream of adequate consistency and of maintaining a stable suspension for a long time. In order to avoid possible interactions with the active ingredients and interference in efficacy tests, no moisturising agents, emollients, antibacterial agents, UV filters and other components typically found in commercial cosmetics were used, although they are known to be ineffective in treating acne. All of these components may possibly be added to the composition of our invention if they are compatible with the stability of the emulsion.

A mixture composed of the following ingredients is used as the basis for the formulation (INCI name in brackets):
about 85% IDS-5 (Cyclopentasiloxane & polysilicone-11)
about 12% DC200cst (dimethicone)
about 3% ascorbic acid.

The desired amounts of active ingredients are added to the basic composition using a turbomixer. The following compositions are prepared:

**Table 1 Composition of formulations for topical use**

| Formulation | Active component |
|---|---|
| A | 2% green tea extract (epigallocatechin-3-gallate 90-95%) |
| B | 2% green tea extract (epigallocatechin-3-gallate 90-95%) |
| | 2% Arctium lappa extract |
| C | 2% green tea extract (epigallocatechin-3-gallate 90-95%) |
| | 2% Arctium lappa extract |
| | 1% S-palmitoyl glutathione |
| D | 2% green tea extract (epigallocatechin-3-gallate 90-95%) |
| | 2% Arctium lappa extract |
| | 1% S-acetyl glutathione |
| E | 2% green tea extract (epigallocatechin-3-gallate 90-95%) |
| | 2% Arctium lappa extract |
| | 1% S-acetyl glutathione |
| | 0.2% salicylic acid |
| F | 2% green tea extract (epigallocatechin-3-gallate 90-95%) |
| | 2% Arctium lappa extract |
| | 1% S-acetyl glutathione |
| | 0.1% sodium phytate |

All indicated percentages are expressed as weight/weight.

### Example 2 Preparation of formulations for oral administration

In two study groups, the topical therapy was combined with oral therapy: in order to study the efficacy of glutathione (or the biological sources thereof), it was decided to administer it orally and not topically. In particular, formulation B (containing EGCG and burdock) was used associating it with the administration of:
N-acetyl cysteine, effervescent 600 mg tablets, or
Glutathione, 500 mg capsules.

The oral administration lasted for 15 weeks, concomitant with topical administration, and was then discontinued: the results are shown in Table 3, Example 4.

### Example 3 Clinical trial protocol

The formulations described in example 1 were tested for efficacy and contraindications on volunteers aged between 14 and 29 years (mean age 18.9 years), of both sexes, suffering from moderate acne. The subjects reporting the use of birth control pills or the use of topical preparations on the face during the test period were excluded, except for the possible use of a light, non-covering cosmetic make-up. All subjects were examined by a dermatologist at the beginning of the test, then every three weeks during the period of application of the formulations, then at the end of the applications (week 15) and three weeks after the end of the test (week 18).

The assessment of the results was carried out by the doctor according to the "global clinical status analysis" methodology, giving each subject a score according to the degree of acne severity according to the IGA (Investigator Global Assessment) table:

**Table 2 Assessment of acne severity level.**

| IGA degree | Condition of the patient (face and neck) |
|---|---|
| 0 | Normal skin, absence of lesions |
| 1 | Presence of rare non-inflammatory lesions and no more than one possible inflammatory lesion |
| 2 | Possible non-inflammatory lesions and presence of rare papules or pustules (mild or very mild acne) |
| 3 | Presence of non-inflammatory lesions, some papules and/or pustules, not more than one nodular lesion (moderate acne) |
| 4 | Presence of more inflammatory and non-inflammatory lesions, presence of more nodular lesions (severe acne) |

### Example 4 Application of topical formulations

Each formulation was tested on a minimum of 8 patients with moderate to severe acne, at least three of whom were of the same sex, by assigning an IGA score to each patient and at each examination; the mean score of the various patients was then calculated for each formulation according to the period of use. At each examination, the subjects were asked to report any inconvenience attributable to the use of the formulation to the doctor. The formulations described in Table 1 were applied independently by the single subjects at least twice daily for a period of 15 consecutive weeks, with the results shown in Table 3. At the 15th week examination, the applications are discontinued, both as to the topical application and the oral administration; the 18th week examination checks the subject's condition after a recovery period at the end of therapy.

The efficacy of the various preparations is assessed according to the reduction in the mean IGA value obtained at week 15 compared with the initial value for the corresponding group of treated subjects (Table 3, delta column 0-15).

**Table 3 Results of the efficacy study in acne therapy.**

| Week | 0 | 3 | 6 | 9 | 12 | 15 | Δ0-15 | 18 | Δ18-15 |
|---|---|---|---|---|---|---|---|---|---|
| Formulati on | Mean IGA index | | | | | | | | |
| A | 3.25 | 2.90 | 2.61 | 2.49 | 2.32 | 2.20 | 1.05 | 2.95 | 0.75 |
| B | 3.18 | 2.88 | 2.57 | 2.24 | 1.99 | 1.79 | 1.39 | 2.75 | 0.96 |
| C | 3.05 | 2.55 | 2.15 | 1.75 | 1.37 | 1.16 | 1.89 | 1.74 | 0.58 |
| D | 3.30 | 2.85 | 2.43 | 1.95 | 1.44 | 1.06 | 2.24 | 1.50 | 0.44 |
| E | 3.15 | 2.70 | 2.28 | 1.93 | 1.55 | 1.20 | 1.95 | 1.70 | 0.50 |
| F | 3.28 | 2.68 | 2.13 | 1.75 | 1.23 | 0.83 | 2.45 | 0.80 | -0.03 |
| B+NAC | 3.32 | 2.87 | 2.27 | 1.94 | 1.49 | 1.04 | 2.28 | 1.12 | 0.08 |
| B+GSH | 3.28 | 2.90 | 2.44 | 1.92 | 1.54 | 1.14 | 2.14 | 1.25 | 0.11 |

Delta 0-15: difference of the initial mean value with respect to the mean IGA value after 15 weeks of application. A greater reduction corresponds to a better effect of the therapy.

Delta 18-15: difference of mean IGA value between week 18 and week 15, i.e., at the end of the application period and after 3 weeks of discontinuation.

As can be seen from the data in Table 3, both the green tea extract (formulation A) and the burdock extract (formulation B, containing both extracts) have a moderate efficacy in reducing acne when applied consistently for 15 weeks (delta column 0-15). At the end of the applications, a resurgence of acne is noted, with an increase in the mean value of inflammation at week 18 compared to week 15 (delta column 18-15).

In addition, it can be noted that the formulations C and D, which comprise both extracts with the addition of a glutathione ester, are surprisingly more effective than formula B, achieving the best reduction in acne at the end of the treatment compared to the mean initial value for the same group of subjects. In particular, the presence of S-acetyl-glutathione associated with EGCG and burdock results to be effective (comparison of formulation D with formulation B, delta column 0-15).

Furthermore, the addition of an exfoliating agent, which further increases the efficacy of the topical formulation, results to be positive: both the combination of salicylic acid (formula E) and of phytic acid (formula F) prove effective, particularly in reducing pustules. Surprisingly, the formulation F proved to be effective for longer: as can be seen from Table 3, column delta18-15, the anti-acne effect still persists after three weeks' of suspension of the applications.

### Example 5 Combined application of topical and oral formulations

In two groups of subjects, the topical application of formulation B (comprising epigallocatechin gallate and burdock) was associated with the oral administration of a source of glutathione, in particular N-acetyl cysteine (NAC, a 600 mg effervescent tablet *per day*) or reduced glutathione (GSH, a 500 mg capsule *per day*)*.* The administration lasted for 15 weeks, the experimental data are shown in Table 3 above.

As can be seen by comparing the data in the last two rows of Table 3 with row B, the *oral* administration of reduced glutathione (row B+GSH) or a biochemical precursor thereof such as N-acetyl cysteine (row B+NAC) is more effective than the topical application of EGCG and burdock alone (row B) in reducing acne manifestations. It can also be seen that the oral administration of glutathione or one of a precursor thereof is a valid alternative to the use of a precursor in the topical formulation: in fact, the reduction in acne obtained by the oral administration is almost equivalent to that obtained by the topical application of S-palmitoyl-glutathione or S-acetyl-glutathione associated with EGCG and burdock (formulations C and D, Table 3).

Furthermore, from the data in Table 3, delta column 18-15, it can be seen that the oral administration of a glutathione source has a longer lasting effect than topical use. In fact, at the 18-week examination, after three weeks of discontinuation of the applications (both the topical and oral formulations), a resurgence of acne was noted, with a moderate increase in the mean IGA value in all study groups except for the subjects on combined topical and oral treatment (rows B+NAC and B+GSH in Table 3) and those treated with the formulation F (comprising phytic acid).

Furthermore, the data surprisingly show that the presence of an exfoliating agent in the topical composition results in a longer lasting effect than the formulation without an exfoliant: both salicylic acid and phytic acid appear to have a prolonged efficacy (Table 3, delta column 18-15, formulations E and F compared to formulation D).

To confirm efficacy, a further group of subjects is now being treated with composition F (Table 1) both topically and orally.

### Example 6 Further topical formulations

In order to verify the possibility of obtaining a stable emulsion, formulations for topical use are prepared containing different proportions of the active ingredients in the same excipients described above. In particular, the formulation F is taken as a reference, varying its composition. The same mixture of excipients as in Example 1 and the same turbomixer apparatus are used; the following formulations are prepared.

**Table 4 Formulations with different concentrations of active ingredients**

| Formulation | Active component |
|---|---|
| F1 | 1% green tea extract (epigallocatechin-3-gallate 90%) |
| | 1% Arctium lappa extract |
| | 0.5% S-acetyl glutathione |
| | 0.1% sodium phytate |
| F2 | 0.5% green tea extract (epigallocatechin-3-gallate 90%) |
| | 0.5% Arctium lappa extract |
| | 0.2% S-acetyl glutathione |
| | 0.1% sodium phytate |
| F3 | 5% green tea extract (epigallocatechin gallate 90%) |
| | 3% Arctium lappa extract |
| | 2% S-acetyl glutathione |
| | 0.3% sodium phytate |
| F4 | 10% green tea extract (epigallocatechin-3-gallate 90%) |
| | 5% Arctium lappa extract |
| | 3% S-acetyl glutathione |
| | 0.3% sodium phytate |

All indicated percentages are expressed as weight/weight.

All formulations F1-F4 prove to be physically stable for a long time: stored at room temperature, no phase separations or other degradations of the emulsion are observed two months after formulation, even in the most concentrated formulations (F3 and F4, Table 4).

### Example 7 Further formulations for oral administration

Capsule formulations are prepared for oral administration containing the three active ingredients of the invention: said oral formulations are intended as a support to local therapy.

**Table 5 Preparations for oral use**

| Capsules | Active component | Dose |
|---|---|---|
| Cap1 | epigallocatechin-3-gallate 95% | 10 mg |
| | *Arctium lappa* extract | 2 mg |
| | reduced glutathione | 100 mg |
| Cap2 | green tea extract (EGCG 80-90%) | 100 mg |
| | *Arctium lappa* extract | 20 mg |
| | reduced glutathione | 100 mg |
| Cap3 | green tea extract (EGCG 80-90%) | 50 mg |
| | *Arctium lappa* extract | 20 mg |
| | N-acetyl cysteine | 200 mg |
| Cap4 | epigallocatechin-3-gallate 95% | 100 mg |
| | *Arctium lappa* extract | 20 mg |
| | N-acetyl cysteine | 300 mg |
| CapS | epigallocatechin gallate 95% | 100 mg |
| | *Arctium lappa* extract | 50 mg |
| | S-acetyl glutathione | 100 mg |

The preparations described above are suitable for the administration of one to three doses per day, in combination with local therapy with the formulations described in the examples 1 and 6.

## Claims

1. Nutraceutical, pharmaceutical or cosmetic composition, comprising:
epigallocatechin-3-gallate in an amount in the range between 0.1% and 10% by weight,
burdock (Arctium lappa) extract in an amount in the range between 0.1% and 10% by weight, and
at least one source of glutathione, **characterised in that** said source of glutathione is different from S-palmitoyl glutathione.

2. The composition according to claim 1, wherein the source of epigallocatechin-3-gallate is an extract of Camellia sinensis.

3. The composition according to claim 1 or 2, wherein said source of glutathione is selected from reduced glutathione, S-acetyl glutathione, N-acetyl cysteine and mixtures thereof.

4. A formulation for topical use comprising the composition of claim 1 and at least one pharmaceutically or cosmetically acceptable excipient.

5. The formulation for use according to claim 4, wherein the concentration of epigallocatechin-3-gallate is in the range between 0.5% and 10%, by weight

6. The formulation for use according to claim 4, wherein the concentration of burdock extract is in the range between 0.5% and 5%, by weight.

7. The formulation for use according to claim 4, wherein said source of glutathione is S-acetyl glutathione in a concentration between 0.1% and 5%, preferably between 0.5% and 3%, by weight.

8. The formulation for use according to claim 4, further comprising an exfoliating agent, **characterized in that** said exfoliating agent is an acid, preferably salicylic acid or phytic acid or their salts or mixture thereof.

9. Kit for the treatment of acne composed of a preparation for topical use and a preparation for oral use, both preparations **characterized by** the fact of containing the composition of claim 1.

10. Kit according to claim 9, further **characterized by** the presence of S-acetyl glutathione in the preparation for topical use and of reduced glutathione, N-acetyl cysteine or mixtures thereof in the oral composition.

11. Kit for the treatment of acne composed of a preparation for topical use having the formulation of claim 8, and a preparation for oral use having the composition of claim 1.

## Patentansprüche

1. Nutrazeutische, pharmazeutische oder kosmetische Zusammensetzung, umfassend:
Epigallocatechin-3-gallat in einer Menge im Bereich zwischen 0,1 und 10 Gew.-%, Klettenextrakt (Arctium lappa) in einer Menge im Bereich zwischen 0,1 und 10 Gew.-%, und zumindest eine Glutathionquelle, **dadurch gekennzeichnet, dass** die Glutathionquelle von S-Palmitoylglutathion verschieden ist.

2. Zusammensetzung gemäß Anspruch 1, wobei die Quelle für Epigallocatechin-3-gallat ein Extrakt von Camellia sinensis ist.

3. Zusammensetzung gemäß Anspruch 1 oder 2, wobei die Glutathionquelle ausgewählt wird aus reduziertem Glutathion, S-Acetylglutathion, N-Acetylcystein und Mischungen davon.

4. Formulierung zur topischen Anwendung, umfassend die Zusammensetzung nach Anspruch 1 und zumindest einen pharmazeutisch oder kosmetisch verträglichen Hilfsstoff.

5. Formulierung zur Verwendung gemäß Anspruch 4, wobei die Konzentration von Epigallocatechin-3-gallat im Bereich zwischen 0,5 und 10 Gew.-% liegt.

6. Formulierung zur Verwendung gemäß Anspruch 4, wobei die Konzentration des Klettenextrakts im Bereich zwischen 0,5 und 5 Gew.-% liegt.

7. Formulierung zur Verwendung gemäß Anspruch 4, wobei die Glutathionquelle S-Acetylglutathion in einer Konzentration zwischen 0,1 und 5 Gew.-%, vorzugsweise zwischen 0,5 und 3 Gew.-%, ist.

8. Formulierung zur Verwendung gemäß Anspruch 4, die ferner ein Peelingmittel umfasst, **dadurch gekennzeichnet, dass** das Peelingmittel eine Säure, vorzugsweise Salicylsäure oder Phytinsäure oder deren Salze oder eine Mischung davon, ist.

9. Kit zur Behandlung von Akne, bestehend aus einem Präparat zur topischen Anwendung und einem Präparat zur oralen Anwendung, wobei beide Präparate **dadurch gekennzeichnet sind, dass** diese die Zusammensetzung gemäß Anspruch 1 enthalten.

10. Kit gemäß Anspruch 9, ferner **gekennzeichnet durch** das Vorliegen von S-Acetylglutathion in dem Präparat zur topischen Anwendung und von reduziertem Glutathion, N-Acetylcystein oder Mischungen davon in der oralen Zusammensetzung.

11. Kit zur Behandlung von Akne, bestehend aus einem Präparat zur topischen Anwendung mit der Formulierung gemäß Anspruch 8 und einem Präparat zur oralen Anwendung mit der Zusammensetzung gemäß Anspruch 1.

## Revendications

1. - Composition nutraceutique, pharmaceutique ou cosmétique, comprenant :
de l'épigallocatéchine-3-gallate dans une quantité dans la plage entre 0,1 % et 10 % en poids,
de l'extrait de bardane (Arctium lappa) dans une quantité dans la plage entre 0,1 % et 10 % en poids, et
au moins une source de glutathion,
**caractérisée par le fait que** ladite source de glutathion est différente du S-palmitoyl glutathion.

2. - Composition selon la revendication 1, dans laquelle la source d'épigallocatéchine-3-gallate est un extrait de Camellia sinensis.

3. - Composition selon l'une des revendications 1 ou 2, dans laquelle ladite source de glutathion est choisie parmi le glutathion réduit, le S-acétyl glutathion, la N-acétyl cystéine et les mélanges de ceux-ci.

4. - Formulation à usage topique comprenant la composition selon la revendication 1 et au moins un excipient pharmaceutiquement ou cosmétiquement acceptable.

5. - Formulation pour une utilisation selon la revendication 4, dans laquelle la concentration d'épigallocatéchine-3-gallate est dans la plage entre 0,5 % et 10 % en poids.

6. - Formulation pour une utilisation selon la revendication 4, dans laquelle la concentration d'extrait de bardane est dans la plage entre 0,5 % et 5 % en poids.

7. - Formulation pour une utilisation selon la revendication 4, dans laquelle ladite source de glutathion est le S-acétyl glutathion dans une concentration entre 0,1 % et 5 %, de préférence entre 0,5 % et 3 %, en poids.

8. - Formulation pour une utilisation selon la revendication 4, comprenant en outre un agent exfoliant, **caractérisée par le fait que** ledit agent exfoliant est un acide, de préférence l'acide salicylique ou l'acide phytique ou leurs sels ou un mélange de ceux-ci.

9. - Kit pour le traitement de l'acné composé d'une préparation à usage topique et d'une préparation à usage oral, les deux préparations étant **caractérisées par** le fait de contenir la composition selon la revendication 1.

10. - Kit selon la revendication 9, **caractérisé en outre par** la présence de S-acétyl glutathion dans la préparation à usage topique et de glutathion réduit, de N-acétyl cystéine ou des mélanges de ceux-ci dans la composition orale.

11. - Kit pour le traitement de l'acné composé d'une préparation à usage topique ayant la formulation de la revendication 8, et d'une préparation à usage oral ayant la composition selon la revendication 1.
